(19)

Europäisches
Patentamt
European
Patent Office
Office européen
des brevets

(11) **EP 3 919 629 A1**

(12) **EUROPEAN PATENT APPLICATION**
published in accordance with Art. 153(4) EPC

(43) Date of publication:
**08.12.2021 Bulletin 2021/49**

(21) Application number: **20863661.3**

(22) Date of filing: **20.08.2020**

(51) Int Cl.:
***C12Q 1/6869*** (2018.01)

(86) International application number:
**PCT/CN2020/110191**

(87) International publication number:
**WO 2021/047363 (18.03.2021 Gazette 2021/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.09.2019 CN 201910863735**

(71) Applicant: **Zhejiang University
Hangzhou, Zhejiang 310058 (CN)**

(72) Inventors:
• **SHU, Qingyao**
  **Hangzhou, Zhejiang 310058 (CN)**
• **WU, Sanling**
  **Hangzhou, Zhejiang 310058 (CN)**
• **TAN, Yuanyuan**
  **Hangzhou, Zhejiang 310058 (CN)**
• **GAO, Qikang**
  **Hangzhou, Zhejiang 310058 (CN)**

(74) Representative: **Wilson Gunn
Blackfriars House
The Parsonage
5th Floor
Manchester M3 2JA (GB)**

(54) **METHOD FOR USING WHOLE GENOME RE-SEQUENCING DATA TO QUICKLY IDENTIFY TRANSGENIC OR GENE EDITING MATERIAL AND INSERTION SITES THEREOF**

(57) Provided is a method for quickly identifying clean transgenic or gene-edited plants and insertion sites by using whole genome re-sequencing data, comprising: extracting genomic DNA; obtaining paired-end sequencing data of a plant whole genome; determining whether an expression vector sequence containing T-DNA sequence, inserted into a plant is known; determining whether transgenic events or gene editing events exist in a plant to be tested, and whether a backbone sequence transfer event occurs; determining the insertion site of the T-DNA sequence. The method combines means of bioinformatic analysis to identify whether there is a transgenic or gene editing event when the expression vector is known or unknown; when the expression vector is known, it not only quickly and accurately provides accurate positioning, direction, copy number and flanking sequence information of the target sequence inserted into genome, but also allows to determine whether there is a backbone sequence inserted into the genome.

FIG.2

EP 3 919 629 A1

SEQUENCE LISTING

<110>  Zhejiang University

<120>  METHOD FOR QUICKLY IDENTIFYING CLEAN TRANSGENIC OR GENE-EDITED
       PLANTS AND INSERTION SITES BY USING WHOLE GENOME RE-SEQUENCING
       DATA

<130>  GWPCTP202105415

<160>  1

<170>  PatentIn version 3.5

<210>  1
<211>  23
<212>  DNA
<213>  Artificial Sequence

<220>
<223>  target sequence  at the third exon of the OsLCT1 gene

<400>  1
cccggcagcg caccgatgtt gct                                            23

**Description**

**CROSS REFERENCE TO RELATED APPLICATIONS**

This patent disclosure claims the priority of Chinese Patent Application No. 201910863735.X entitled "Method for quickly identifying clean transgenic or gene-edited plants and insertion sites by using whole genome re-sequencing data" filed with the China National Intellectual Property Administration on September 12, 2019, the disclosure of which is incorporated by reference herein in its entirety as part of the present application.

**TECHNICAL FIELD**

The disclosure relates to the technical field of bioinformatics, in particular to a method for quickly identifying clean transgenic or gene-edited plants and insertion sites by using whole genome re-sequencing data.

**BACKGROUND ART**

The method of rapid identification of clean transgenic or gene-edited plants and their insertion sites refers to a technology that uses transgenic or gene editing technology to "edit" or transform the target genes in animals and plants based on the principle of genetic engineering, according to people's wishes, so as to realize the directed transformation of biological genetic traits, for example, knockout and addition of specific DNA fragments.

The traditional methods for detecting transgenic or exogenous gene fragments mainly include: 1. in situ hybridization to verify the integration, integrity and approximate gene copy number; 2. real-time fluorescence quantitative PCR to accurately calculate the copy number of transgenes; 3. chromosome walking technology to confirm the precise insertion site of transgene; 4. fluorescence in situ hybridization to prove the chromosome position of transgene integration.

However, these methods have some shortcomings such as long experimental period, single identification item and low throughput. With the development of sequencing technology, the optimization of its price, and the popularization of transgenic or gene editing technology, the methods for quickly identifying the insertion sites of transgenic or gene editing events and whether there are insertions of non-target fragments by using whole genome re-sequencing data are becoming more and more important.

Patent with issue number CN103270175B discloses a method and a system for detecting insertion sites of transgenic exogenous fragments, which comprises: determining exogenous one-sided short fragments and genomic one-sided short fragments by comparison, and determining the insertion sites of exogenous fragments in genome sequences according to the intersection of exogenous one-sided short fragments and genomic one-sided short fragments.

Patent with issue number CN105631242B discloses a method for quickly identifying transgenic events by using whole genome re-sequencing data, which combines means of bioinformatic analysis to identify transgenic events by mapping the sequencing reads to genome and expression vector sequences respectively, and identifies the position, direction, copy number and flanking sequence information of exogenous fragments inserted into the genome by a three-step positioning method, as well as the homozygous and heterozygous states of transgenic samples.

However, none of the above patents considers the recombination or rearrangement of the sequences around the insertion site caused by the insertion of exogenous sequences, which further reduces the sensitivity of the method and raises the probability of false negative and missed detection. The above patents also do not take into account the increase in false positives when the vector sequence is partially homologous to the host genome sequence. In addition, the above methods all need to map the reads to the wild-type genome sequence of plants to be tested, which is tedious, time-consuming and laborious.

**SUMMARY OF THE INVENTION**

The disclosure provides a method for quickly identifying clean transgenic or gene-edited plants and insertion sites by using whole genome re-sequencing data, which combines means of bioinformatics analysis to identify whether there is a transgenic or gene editing event when the expression vector is known or unknown. When the expression vector is known, the method can not only quickly and accurately provide the accurate positioning, direction, copy number and flanking sequence information of the target sequence inserted into genome, but also allows to determine whether there is a backbone sequence, which is a sequence other than the target sequence, inserted into the genome, and provide the same positioning.

The specific technical scheme is as follows.

A method for using whole genome re-sequencing data to quickly identify clean transgenic or gene-edited plants and insertion sites thereof, comprising:

(1) extracting genomic DNA of plant samples to be tested after being processed by transgenic or gene editing technology;

(2) carrying out whole genome re-sequencing on the genome DNA to obtain paired-end sequencing data of the whole genome;

(3) determining whether an expression vector sequence containing T-DNA sequence, inserted in plants to be tested, is known or not, and performing the following operations to obtain the determination result:

> if the expression vector sequence inserted in the plant to be detected is known, the expression vector containing T-DNA sequence is used as a reference sequence, the paired-end sequencing data after quality control were aligned to the known plasmid DNA to obtain a mapping database;
> if the expression vector sequence inserted in the plant to be detected is unknown, a generic pan-vector library is used as a reference sequence, the paired-end sequencing data after quality control were aligned to the sequences from pan-vector database to obtain a mapping database;

(4) counting the read numbers of matching expression vector sequences or matching pan-vector library in the mapping database, the read numbers matching backbone sequences, the base coverage and average sequencing depth of T-DNA sequences, the average sequencing depth of genome of plant samples to be tested, the sequence length of expression vectors and the length of sequencing read, respectively, to determine whether transgenic events or gene editing events exist in plants to be tested, whether backbone sequence transfer events occur, and the copy number of inserted sequences according to the following formula;

the criteria for determining whether there are transgenic events or gene editing events are:

> (4-1) if the expression vector sequence is known:
>
> > $VRN \geq Gdepth/2 + VectorLen/ReadLen$, and $Tcov \geq 0.9$;
> > wherein, VRN represents the number of reads that matched the vector; Gdepth represents the average sequencing depth of the genome of plants to be tested; VectorLen represents the sequence length of the expression vector; ReadLen represents the length of sequencing read; Tcov represents sequencing coverage of T-DNA sequence, Tdepth represents average sequencing depth of T-DNA sequence, and Bdepth represents average sequencing depth of backbone sequence;
>
> (4-2) if the expression vector sequence is unknown:
>
> > $FRN \geq (Gdepth/2) \times 10$, combined with characteristic structure ie., CaMV35S or OsCas9 was identified;
> > wherein, FRN represents the number of reads matching the generic pan-vector database; Gdepth represents the average sequencing depth of the genome of plants to be tested;
> > the criterion for determining whether there is backbone sequence transfer event is: $BRN \geq Gdepth/3$;
> > wherein, BRN represents the number of reads matching the backbone sequence; Gdepth represents the average sequencing depth of the genome of plants to be tested;
>
> (4-3) determining copy number: the copy number of inserted T-DNA = Tdepth/Gdepth; the copy number of inserted backbone sequences = Bdepth/Gdepth;
> Tdepth represents the average sequencing depth of T-DNA sequence, Gdepth represents the average sequencing depth of the genome of plants to be tested, and Bdepth represents the average sequencing depth of the backbone sequence;

(5) taking the expression vector containing T-DNA sequence as a reference sequence, according to the data of the mapping database, extracting a qualified paired-end reads, wherein the paired-end reads must contain one single-end read I which completely matches the expression vector sequence and the other single-end read II which does not match or does not completely match the expression vector sequence; then the single-end read II is compared with the expression vector sequence containing T-DNA sequence and the wild-type genome sequence of plants to be tested for local homology analysis; the insertion site of T-DNA sequence is determined according to the following different cases,

> (5-1) if one end of the single-end read II can match the expression vector sequence, the other end can match the wild-type genome sequence, and there are at least three single-end reads II with the same matching starting position of genome or expression vector, then it is determined that there is a candidate insertion site on the single-end read II, and the breakpoint is at the integrated site.;

(5-2) if the single-end read II cannot match the expression vector sequence, but can match the wild-type genome sequence, and there are at least three single-end reads II with the same matching starting position of the genome, then it is determined that there is an insertion site on the single-end read II, and the starting position matching the genome is the insertion site;

(5-3) if the single-end read II matches neither the expression vector sequence nor the wild-type plant genome, then it is necessary to assemble the paired-end reads corresponding to the single-end read II into a fragment according to the sequence overlapping characteristics, and ligate the fragment with the T-DNA sequence or inserted expression vector fragment with N, with the ligated sequence being used as reference sequence, repeating steps (3) and (5) until the single-end read II can match the wild-type genome sequence of plants to be tested and can be determined by step (5-1) or (5-2).

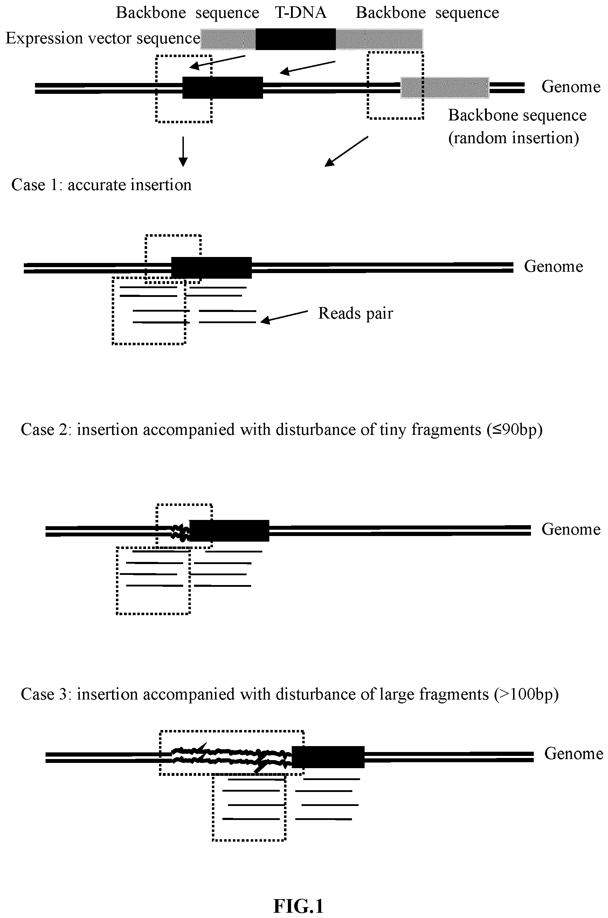

As shown in Fig. 1, an expression vector sequence includes both a target T-DNA sequence and a sequence other than target T-DNA, which we define as backbone sequence. Whether T-DNA sequence or backbone sequence is inserted into the wild-type genome of plants to be tested, there are three situations: the first situation is precise insertion, that is, the foreign fragment is seamlessly inserted into the plant genome without any mutations near the insertion sites. As for the case where one single-end read I that matches the expression vector, the other single-end read II that does not match or does not completely match the expression vector sequence has two types, one is the read (can be located by 5-1) that spans the "breakpoint" between the inserted sequence and the genome sequence, the other is read (can be located by 5-2) completely derived from the genome sequence. The second situation is insertion accompanied with insertion or rearrangement of tiny fragments. Here, tiny fragments are defined as fragments less than or equal to 90bp (here, the length may vary) according to the read length and sensitivity of the method. The single-end read II here also has two types, one is the read (can be located by 5-1) that spans the "breakpoint" between the inserted sequence and the genome sequence, the other is read (can be located by 5-2) completely derived from the genome sequence. The third situation is insertion accompanied with insertion or rearrangement of large fragments. Here, large fragments are defined as fragments more than 100bp (here, the length may vary) according to the read length and sensitivity of the method. The single-end read II here also has two types, one is the read (can be located by 5-1) that spans the "breakpoint" between the inserted fragments and the rearranged sequence, the other is read completely derived from the rearranged sequence, both of which can be used for 5-3 positioning.

In some embodiments, in step (2), the whole genome re-sequencing of the genomic DNA is carried out, and quality control on the raw sequencing data is performed to obtain paired-end sequencing data of the whole genome after quality control;

the quality control comprises the following steps:

(2-a) removing the adapters of sequencing reads;
(2-b) removing the read with the ratio of N greater than 20%;
(2-c) removing the paired-end reads corresponding to the single-end read when the number of low-quality bases contained at the 3' end of the single-end read exceeds one third of the length of the read; the low-quality base is the base having a quality value less than or equal to 20.

In some embodiments, in step (3), the generic pan-vector library is a complete vector sequence database as complete as possible collected from the public database (NCBI, https://www.ncbi.nlm.nih.gov/).

In some embodiments, in step (5-1), the interval sequence in the single-end read II that matches the expression vector sequence is compared with the wild-type genome sequence of the plant samples to be tested;

if it is not a homologous sequence, then it is determined that the candidate insertion site on the single-end read II is a real insertion site; if it is a homologous sequence, then it is determined that the candidate insertion site on the single-end read II is a false positive insertion site.

In some embodiments, in step (5-2), if the single-end read II is the left-end sequence of the paired-end reads, then the determined insertion site is the maximum of the sites on single-end read II ; if the single-end read II is the right-end sequence of the paired-end reads, then the determined insertion site is the minimum of the sites on the single-end read II.

In some embodiments, in steps (5-1)-(5-3), the matching criteria are: base similarity ≥95%, number of mismatched bases ≤5, and number of base gaps ≤5.

In some embodiments, the method further comprises step (6): designing pre-primer and post-primer sequences according to the insertion sites obtained in step (5), and verifying the integrated sites by PCR technology.

Compared with the prior arts, the disclosure has the following beneficial effects:

(1) The method of the disclosure combines bioinformatics analysis means to identify whether transgenes or gene editing events occur under the condition that the expression vector is known or unknown. When the expression vector is known, it not only quickly and accurately provides accurate positioning, direction, copy number and flanking sequence information of the target sequence inserted into the genome, but also allows to determine whether there is a backbone sequence, which is a sequence other than the target sequence, inserted into the genome, and provide the same positioning.

(2) The method of the disclosure not only considers the recombination or disorder of the sequences around the genomic insertion site caused by the insertion of exogenous sequences, which further reduces the sensitivity of the method and raises the false negative and probability of missed detections, but also considers the increase in false positives when the vector sequence is partially homologous to the genome sequence.

(3) Compared with the traditional experimental method, the method has the advantages of short time consumption, economy, repeatability and the like, and it can be used to determine the influence of transgenic or gene editing events on plant genomes in a short time, which is beneficial to the safety risk assessment of transgenic plants, and promotes the application of transgenic or gene editing technologies in agriculture.

## BRIEF DESCRIPTION OF THE DRAWINGS

Fig. 1 is a schematic diagram for the principle of the method of the present disclosure for quickly identifying clean transgenic or gene-edited plants and insertion sites by using whole genome re-sequencing data;

wherein, an expression vector sequence includes both a target T-DNA sequence and a sequence other than target T-DNA, which we define as backbone sequences. Whether T-DNA sequence or backbone sequence is inserted into the wild-type genome of plants to be tested, there are three situations: the first situation is precise insertion, that is, the foreign fragment is seamlessly inserted into the plant genome without any mutations near the insertion sites. As for the case where one single-end read I that matches the expression vector sequence, the other single-end read II that does not match or does not completely match the expression vector sequence has two types, one is the read (can be located by 5-1) that spans the "breakpoint" between the inserted sequence and the genome sequence, the other is read (can be located by 5-2) completely derived from the genome sequence. The second situation is insertion accompanied with insertion or rearrangement of tiny fragments, here, tiny fragments are defined as fragments less than or equal to 90bp (here, the length may vary) according to the length of read and sensitivity of the method, the single-end read II here also has two types, one is the read (can be located by 5-1) that spans the "breakpoint" between the inserted sequence and the genome sequence, the other is read (can be located by 5-2) completely derived from the genome sequence. The third situation is insertion accompanied with insertion or rearrangement of large fragments. Here, large fragments are defined as more than 100bp (here, the length may vary) according to the length of read and sensitivity of the method. The single-end read II here also has two types, one is the read (can be located by 5-1) that spans the "breakpoint" between the inserted sequence and the rearranged sequence, the other is read completely derived from the rearranged sequence, both of which can be used for 5-3 positioning.

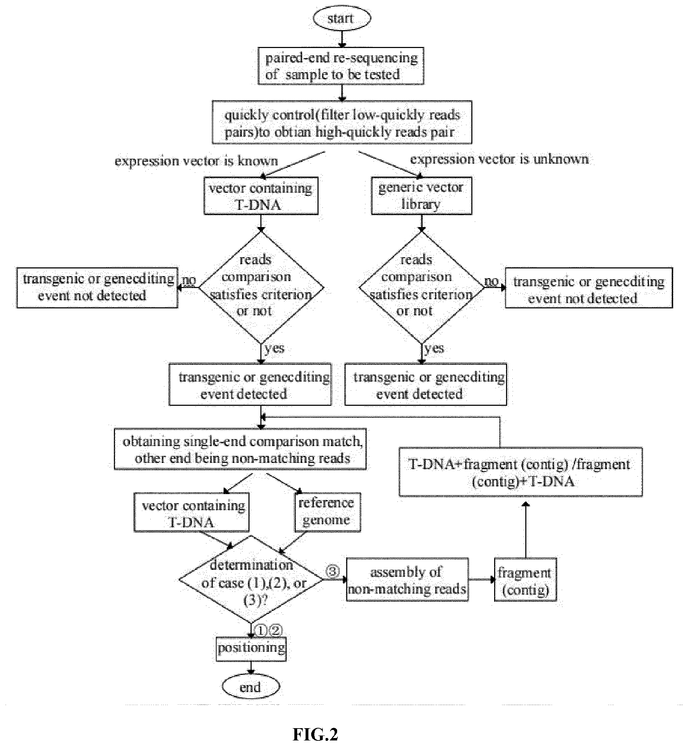

Fig. 2 is a schematic flow chart of the method for f the present disclosure quickly identifying clean transgenic or gene-edited plants and insertion sites by using whole genome re-sequencing data.

Fig. 3 is a schematic diagram for judging whether there are transgenic or gene editing events, backbone sequence insertion events and copy number in Example 1;

Wherein, the outermost layer of the graph is the length scale of the expression vector, the second layer from outside to inside is the structural annotation of the expression vector sequence, the third layer from outside to inside is the coverage area of the vector DNA measured by the whole genome sequencing in plant, and the fourth layer is the times of sequencing to each base of expression vector sequence, that is, the sequencing depth. It can be seen from the figure that there are not only T-DNA sequences but also backbone sequences inserted in the genome.

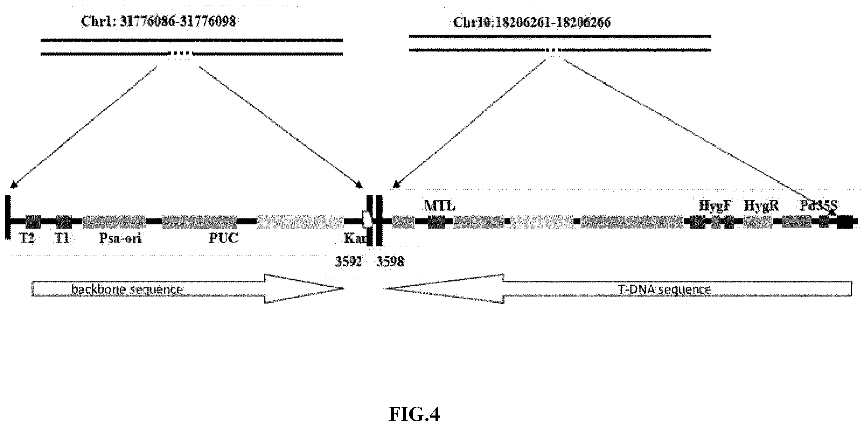

Fig. 4 is a schematic diagram of the specific condition that the expression vector is inserted into the genome in the rice plants to be tested in Example 1.

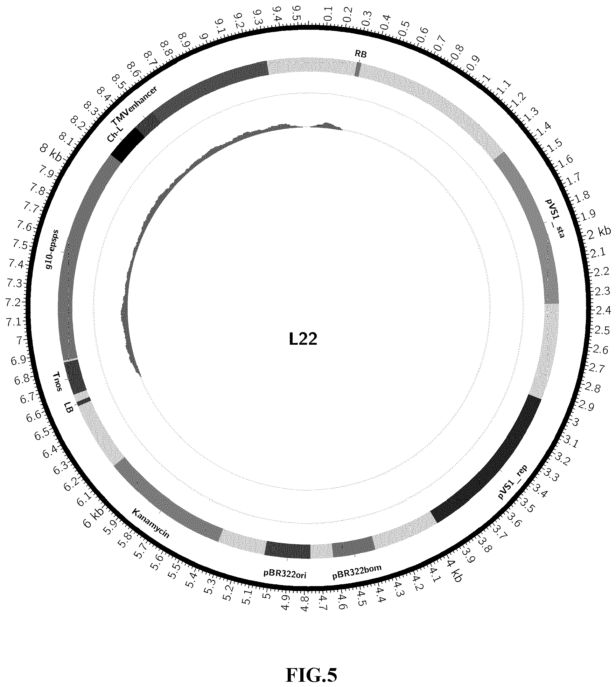

Fig. 5 is a schematic diagram for judging whether there are transgenic or gene editing events, backbone sequence insertion events and copy number in Example 2.

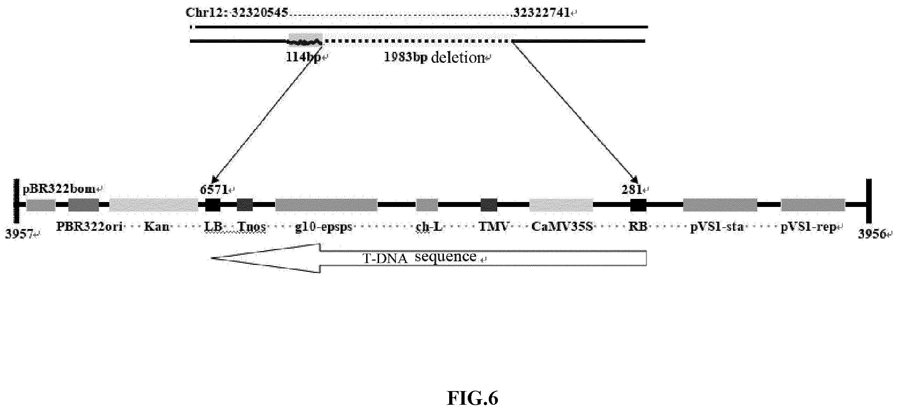

Fig. 6 is a schematic diagram of the specific condition that the expression vector is inserted into the genome in the soybean plants to be tested in Example 2.

## DETAILED DESCRIPTION OF THE EMBODIMENTS

The present disclosure will be further explained with specific examples below. These examples are only used to illustrate the disclosure and are not meant to limit the scope of the disclosure. The experimental methods without specific conditions indicated in the examples are carried out according to the conventional conditions or the conditions described in Molecular Cloning: A Laboratory Manual, edited by J.Sambrook and D.W.Russell, translated by Huang Peitang et al., published by Science Press, 2002.08, 3rd edition.

Terms involved in the present disclosure are defined as follows:

Read: each sequence of "ATCG" permutation and combination obtained by the sequencer.

Paired-end reads: the genome is randomly broken into DNA fragments with different lengths by ultrasonic instrument, the DNA fragments with fixed size are recovered and purified by PCR, the two ends of these DNA fragments are fixed on the substrate by adding adapters, and then the DNA fragments are sequenced from the two ends to the middle of the fragments to obtain a paired-end reads from the same DNA fragment.

Single-end read: compared with paired-end reads, it refers to any read in a paired-end reads.

Average sequencing depth: the ratio of total bases obtained from sequencing interval to sequence interval length; for average sequencing depth of T-DNA and backbone sequences, the ratio of total bases obtained from sequencing interval of T-DNA and backbone, respectively (excluding homologous interval with genome) to sequencing interval (excluding homologous interval with genome) length.

Base coverage of T-DNA sequence: the ratio of the length of T-DNA interval measured by sequencing to the length of T-DNA sequence.

Complete match: for a 150bp read, the sequence can be completely matched with the reference sequence, allowing 2-3 base mismatches and gaps less than 10bp.

Incomplete match: for a 150bp read, there are sequences less than or equal to 90bp that can match the reference sequence.

**EXAMPLE 1**

A method for quickly identifying clean gene-edited plants and insertion sites thereof by using whole genome re-sequencing data, which comprises:

(1) extraction of genomic DNA from experimental materials and plants to be tested: the rice gene-edited sample R07 used pHUN4c12S as a vector and had a total length of 12,314bp. The target gene for gene editing was *MTL* (*LOC_Os03g27610*), which encoded a phospholipase.

Firstly, a target sequence (20nt) was designed in the third exon of the *MTL* gene, and the target sequence was integrated into the vector pHUN4c12S to obtain the pRUN4c12S-*MTL* plasmid. Then, the plasmid was transformed into Agrobacterium strain. Finally, pHUN4c12S-*MTL* was transformed into japonica rice variety Xidao No.1 by Agrobacterium-mediated transgenic method. After screening, differentiation and rooting, the To transgenic regenerated seedlings were obtained, and the $T_1$ plant R07 was obtained by continuous planting. After transplanting plants to be tested to the field for about 30 days, the leaves were picked, the genomic DNA of plants to be tested were extracted with conventional DNA extraction kit, and whole genome re-sequencing was performed. The reference genome sequence of rice was RGAP v7 (http://rice.plantbiology.msu.edu/annotation_pseudo.shtml).

(2) The whole genome re-sequencing was carried out on the genome DNA of plants to be tested, and quality control was carried out on the raw sequencing data to obtain clean paired-end sequencing data of the whole genome;

the whole genome paired-end sequencing was performed on the gene-edited rice to be tested, transformed with pHUN4c12S by high-throughput sequencer (Illumina Sequencer, Beijing Biomarker Technologies Co., Ltd.), the insertion size was 300bp and the read length was 150bp, 48G raw sequencing data was obtained, and the average sequencing depth was 57 layers.

The raw sequencing data was quality controlled, the software used for quality control was Trimmomatic, version 0.36, and criteria of the quality control were:

(2-a) the adapters of the sequencing reads were removed;

(2-b) the read with the ratio of N greater than 20% was removed;

(2-c) when the number of low-quality bases (base mass ≤20) contained at the 3' end of a single-end read (i.e. one of the paired-end reads) exceeded one third of the length of the read, this paired-end reads was removed; parameters were set to tailing: 20, MINLEN: 100, and high quality data after quality control was obtained.

(3) Whether an expression vector sequence containing T-DNA sequence, inserted in plants to be tested, was known or not was judged, and the following operations based on the judgment result were performed:

if the expression vector sequence inserted in the plant to be detected was known, then the expression vector containing T-DNA sequence was used as a template and mapped to the paired-end sequencing data to obtain a mapping database;

if the expression vector sequence inserted in the plant to be detected was unknown, then a generic vector library was used as a template and mapped to the paired-end sequencing data to obtain a mapping database;

the judgment result was that the expression vector sequence inserted in plants to be tested was known, thus the expression vector pHUN4c12S-*MTL* containing T-DNA sequence was used as a reference sequence, and the clean paired-end sequencing data after quality control was mapped and analyzed with the reference sequence to obtain a mapping database.

The specific mapping analysis steps were as follows: the software used was bowtie2, version 2.2.1, the default parameters (the mapping standard had been defined) were adopted, the obtained alignment result information file was in a SAM format, the SAM format file was converted into binary bam format file (i.e. mapping database), and the conversion tool was samtools. In order to reduce the interference of sequencing repetitive sequences (two identical sequences matching the same position and direction of the expression vector) on the subsequent localization results, the step of removing repetitive sequence alignment results was introduced in the format conversion process. The tool used to remove repetitive sequences was the subroutine module MarkDuplicates.jar in picard software, the parameter REMOVE_DUPLICATES was set to true, and other parameters were set to default.

(4) The read number (VRN) of the matching expression vector sequence, the read number (BRN) of the matching backbone sequence, the base coverage (TCov) and the average sequencing depth (Tdepth) of the T-DNA sequence, the average sequencing depth (Gdepth) of the sample genome, the sequence length (VectorLen) of the expression vector, the read length (ReadLen) were counted, respectively, and whether transgenic events or gene editing events exist in plants to be tested, whether backbone sequence transfer events occur were judged according to the following formula;

the criteria for judging whether there were transgenic events or gene editing events were:

$VRN \geq Gdepth/2+VectorLen/ReadLen$, and $Tcov \geq 0.9$;

wherein, VRN represented the number of reads matching the expression vector sequence; Gdepth represented the average sequencing depth of the genome of plants to be tested; VectorLen represented the sequence length of the expression vector; ReadLen represented the length of read; Tcov represented base coverage of T-DNA sequence;

the criterion for judging whether there was backbone sequence transfer event was: $BRN \geq Gdepth/3$;

wherein, BRN represented the number of reads matching the matching backbone sequence; Gdepth represented the average sequencing depth of the genome of plants to be tested.

The results showed that the read number (VRN) of the matching expression vector sequence was 2725, the read number (BRN) of the matching backbone sequence was 1053, the base coverage (TCov) of the T-DNA sequence was 0.997, the average sequencing depth (Tdepth) of the T-DNA sequence was 23 layers, the average sequencing depth of backbone sequences was 27 layers, the average sequencing depth (Gdepth) of the sample genome was 57 layers, the sequence length (VectorLen) of the expression vector was 12314 bp, the read length (ReadLen) was 150 bp.

(4-1) $VRN \geq 111 = (57/2+12314/150)$; $Tcov \geq 0.9$;

(4-2) $BRN \geq 19 = 57/3$;

(4-3) determination of copy number: the copy number of inserted T-DNA= $23/57 \approx 1/2$; the copy number of inserted backbone sequences= $27/57 \approx 1/2$;

Based on the above judgment criteria, it was judged that there were transgenic or gene editing events in the samples, and there were backbone sequence transfer events, which were heterozygote..

(5) The expression vector containing T-DNA sequence was taken as a reference sequence, according to the data of the mapping database (bam format file), a qualified paired-end reads was extracted through samtools software, wherein the paired-end reads must contain one single-end read I which completely matched the expression vector sequence and the other single-end read II which did not match or did not completely match the expression vector sequence; then the single-end read II was compared with the expression vector sequence containing T-DNA sequence and the wild-type genome sequence of plants to be tested for local homology analysis; the local homology comparison analysis software was blast, 2.2.28+ version, and the outfmt parameter was set to 6 to ensure that the output result was in m8 format separated by tab key, which was convenient for subsequent processing, the num_descriptions parameter or max_target_seqs parameter was set to 1, and the evalue parameter was set to $1e^{-5}$. The insertion site of T-DNA sequence was determined based on the following different cases:

(5-1) if one end of the single-end read II could match the expression vector sequence, the other end could match the wild-type genome sequence, and there were at least three single-end reads II with the same matching starting position of genome or expression vector, then it was determined that there was a candidate insertion site on the single-end read II, and the read II interval segment matching the expression vector sequence was extracted and compared with the wild-type genome of the transgenic sample to be tested. If there was no homologous sequence, then the genome matching position nearest to the position of the expression vector sequence was the true insertion site, if there was a homologous sequence, then it was determined that the candidate insertion site on the single-end read II was a false positive insertion site. The matching criteria were: base similarity $\geq 95\%$, mismatched bases $\leq 5$, base gaps $\leq 5$.

(5-2) If the single-end read II could not match the expression vector sequence, but could match the wild-type genome sequence, and there were at least three single-end reads II with the same matching starting position of the genome, then it was determined that there was an insertion site on the single-end read II, and the starting position matching the genome was the insertion site; the matching criteria were: base similarity $\geq 95\%$, mismatched bases $\leq 5$, base gaps $\leq 5$.

If the single-end read II was the left-end sequence of the paired-end reads, then the insertion site on the determined single-end read II was the maximum site; if the single-end read II was the right-end sequence of the paired-end reads, then the insertion site in the single-end read II was the minimum site.

(5-3) if the single-end read II could not match either the expression vector sequence or the wild-type genome sequence, then it was necessary to assemble the paired-end reads corresponding to the single-end read II into a fragment according to the sequence overlapping characteristics, and ligate the fragment to the T-DNA sequence or inserted expression vector fragment with N to be used as reference sequence, and steps (3) and (5) were repeated until the single-end read II could match the wild-type genome sequence of plants to be tested, and could be determined by step (5-1) or (5-2).

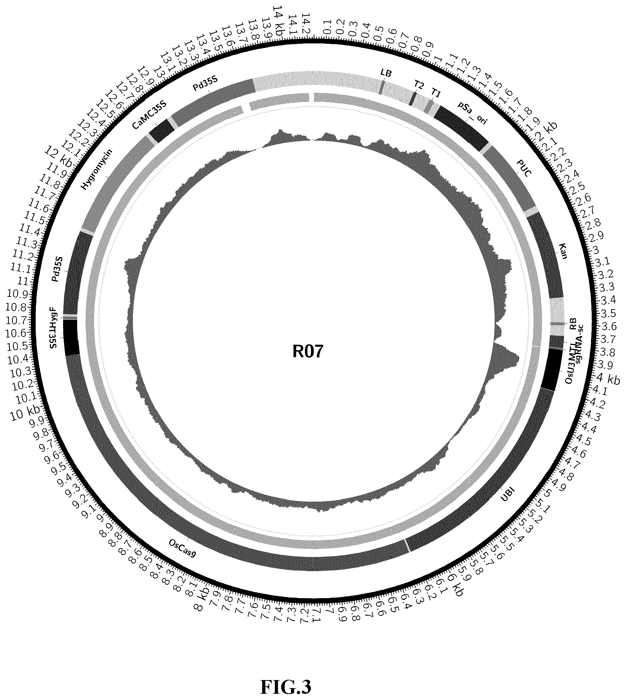

According to the method of step (5), the determination result is shown in Table 1, and the genome schematic diagram of inserted T-DNA sequence is shown in Fig. 3 and Fig. 4.

It can be seen from Fig. 3 and Fig. 4 that this Example belongs to the case of step (5-1), and the insertion site is located by matching the single-end read II with the expression vector sequence and the wild-type genome sequence.

Table 1 Final output results of positioning of rice plants to be tested

| Sample | R07 | R07 | R07 | R07 |
|---|---|---|---|---|
| Vector name | pHUN4c12S-*MTL* | pHUN4c12S-*MTL* | pHUN4c12S-*MTL* | pHUN4c12S-*MTL* |
| Vector length | 14251 | 14251 | 14251 | 14251 |
| Insertion interval | Backbone sequence | Backbone sequence | T-DNA | T-DNA |
| Insertion direction | - | + | - | + |
| Insertion sites | 3592(right-end of backbone) | 660(left-end of backbone) | 649(left-end of T-DNA) | 3598(right-end of T-DNA) |
| Species genome | O_sativa_v7.0 | O_sativa_v7.0 | O_sativa_v7.0 | O_sativa_v7.0 |
| Location chromosome | Chr1 | Chr1 | Chr10 | Chr10 |
| Chromosome length | 43270923 | 43270923 | 23207287 | 23207287 |
| Genome insertion position | 31776098 | 31776086 | 18206261 | 18206265 |
| End position | 31776598 | 31775586 | 18205761 | 18206765 |
| Genome chain direction | + | - | - | + |

## EXAMPLE 2

(1) Extraction of genomic DNA from experimental materials and plants to be tested: the transgenic soybean sample L22 used pSOY19 as a vector and had a total length of 9557bp. The target gene contained in the vector was g10-epsps, which was a glyphosate-tolerant gene.

According to the g10-epsps gene sequence, the target sequence was integrated into the vector pSOY19 to obtain the pSOY19-g10-epsps expression plasmid vector. Then the plasmid was transformed into Agrobacterium strain. Finally, pSOY19-g10-epsps was transformed into Huachun No.3 by Agrobacterium-mediated transgenic method. After screening, differentiation and rooting, the transgenic soybean L22 was obtained.

After the strains were cultivated regularly for 100 days, the leaves were picked, the genomic DNA of plants to be tested were extract by the conventional DNA extraction kit, and the whole genome re-sequencing was performed. The reference genome sequence of soybean was the cultivar Williams 82 sequence Gmax_275_v2.fa, and the download address is https://phytozome.jgi.doe.gov/pz/portal.html.

(2) The whole genome re-sequencing was carried out on the genome DNA of plants to be tested, and quality control was carried out on the raw sequencing data to obtain paired-end sequencing data of the whole genome;

the whole genome paired-end sequencing was performed on the transgenic soybean samples to be tested, transformed with pSOY19 by high-throughput sequencer (Illumina XTen, Novogene Technology Co., Ltd.), the insertion size was 300bp and the read length was 150bp, 52G raw sequencing data was obtained, and the average sequencing depth was 23 layers.

The raw sequencing data was quality controlled, the software used for quality control was Trimmomatic, version 0.36, and criteria of the quality control were:

(2-a) The adapters of reads were removed;

(2-b) The read with the ratio of N greater than 20% was removed;

(2-c) When the number of low-quality bases (base quality score ≤20) contained at the 3' end of a single-end read (i.e. one of the paired-end reads) exceeded one third of the length of the read, the paired-end reads was removed; parameters were set to tailing: 20, MINLEN: 100, and high quality data after quality control was obtained.

(3) Whether an expression vector sequence containing T-DNA sequence, inserted in plants to be tested, was known or not was determined, and the following operations according to the determination result were performed:

if the expression vector sequence inserted in the plant to be detected was known, the expression vector containing T-DNA sequence was used as a reference sequence, and the paired-end sequencing data were mapped to the reference sequence to obtain a mapping database;

if the expression vector sequence inserted in the plant to be detected was unknown, a generic vector library was used as reference sequences and the paired-end sequencing data were mapped to the reference sequences to obtain a mapping database;

the judgment result was that the expression vector sequence inserted in plants to be tested was known, thus the expression vector pSOY19-g10-epsps containing T-DNA sequence was used as a reference sequence, and the clean paired-end sequencing data after quality control was mapped and analyzed with the reference sequence to obtain a mapping database.

The specific mapping analysis steps were as follows: the software used was bowtie2, version 2.2.1, the default parameters (the mapping standard had been defined) were adopted, the obtained comparison result information file was in SAM format, the SAM format file was converted into binary bam format file (i.e. mapping database), and the conversion tool was samtools. In order to reduce the interference of sequencing repetitive sequences (two identical sequences matching the same position and direction of the expression vector) on the subsequent localization results, the step of removing repetitive sequence alignment results was introduced in the format conversion process.

The tool used to remove repetitive sequences was the subroutine module MarkDuplicates.jar in picard software, the parameter REMOVE_DUPLICATES was set to true, and other parameters were set to default.

(4) The read number (VRN) of the matching expression vector sequence, the read number (BRN) of the matching backbone sequence, the base coverage (TCov) and the average sequencing depth (Tdepth) of the T-DNA sequence, the average sequencing depth (Gdepth) of the sample genome, the sequence length (VectorLen) of the expression vector, the read length (ReadLen) were counted, respectively, and whether transgenic events or gene editing events exist in plants to be tested, whether backbone sequence transfer events occur were determined according to the following formula;

the criteria for judging whether there were transgenic events or gene editing events were:

$$VRN \geq Gdepth/2 + VectorLen/ReadLen,$$

and Tcov ≥ 0.9;

wherein, VRN represented the number of reads matching the expression vector sequence; Gdepth represented the average sequencing depth of the genome of plants to be tested; VectorLen represented the sequence length of the expression vector; ReadLen represented the length of read; Tcov represented base coverage of T-DNA sequence; the criterion for judging whether there was backbone sequence transfer event was: BRN ≥ Gdepth/3; wherein, BRN represented the number of reads matching the matching backbone sequence; Gdepth represented the average sequencing depth of the genome of plants to be tested;

The results showed that the read number (VRN) of the matching expression vector sequence was 293, the read number (BRN) of the matching backbone sequence was 0, the base coverage (TCov) of the T-DNA sequence was 0.986, the average sequencing depth (Tdepth) of the T-DNA sequence was 14 layers, the average sequencing depth (Gdepth) of the sample genome was 23 layers, the sequence length (VectorLen) of the expression vector was 9557 bp, the read length (ReadLen) in the mapping database was 150 bp.

$$(4\text{-}1)\ \mathrm{VRN} \geq 75 = (23/2 + 9557/150);$$

Tcov ≥ 0.9;

$$(4\text{-}2)\ \mathrm{BRN} < 8 \approx 23/3;$$

(4-3) determination of copy number: the copy number of inserted T-DNA = 14/23≈1/2, heterozygote.

According to the above determination criteria, it was determined that there were transgenic or gene editing events in the samples, the events were heterozygote, but there were no backbone sequence transfer events.

(5) The expression vector containing T-DNA sequence was taken as a reference sequence, according to the data of the mapping database (bam format file), a qualified paired-end reads was extracted through samtools software, wherein the paired-end reads must contain one single-end read I which completely matched the expression vector sequence and another single-end read II which did not match or did not completely match the expression vector sequence; then the single-end read II was compared with the expression vector sequence containing T-DNA sequence and the wild-type genome sequence of plants to be tested for local homology analysis. The local homology comparison analysis software was blast, 2.2.28+ version, and the outfmt parameter was set to 6 to ensure that the output result was in m8 format separated by tab key, which was convenient for subsequent processing, the num_descriptions parameter or max_target_seqs parameter was set to 1, and the evalue parameter was set to $1e^{-5}$.

The insertion site of T-DNA sequence was determined according to the following different cases:

(5-1) if one end of the single-end read II could match the expression vector sequence, the other end could match the wild-type genome sequence, and there were at least three single-end reads II with the same matching starting position of genome or expression vector, then it was determined that there was a candidate insertion site on the single-end read II, and the read II interval segment matching the expression vector sequence was extracted and compared with the wild-type genome of the transgenic sample to be tested. If there was no homologous sequence, then the genome matching position nearest to the position of the expression vector sequence was the true insertion site, if there was a homologous sequence, then it was determined that the candidate insertion site on the single-end read II was a false positive insertion site. The matching criteria were: base similarity ≥ 95%, mismatched bases ≤ 5, base gaps ≤ 5.

(5-2) if the single-end read II could not match the expression vector sequence, but could match the wild-type genome sequence, and there were at least three single-end reads II with the same matching starting position of the genome, then it was determined that there was an insertion site on the single-end read II, and the starting position matching the genome was the insertion site; the matching criteria were: base similarity ≥ 95%, mismatched bases ≤ 5, base gaps ≤ 5.

If the single-end read II was the left-end sequence of the paired-end reads, the insertion site on the determined single-end read II was the maximum site. If the single-end read II was the right-end sequence of the paired-end reads, the insertion site in the single-end read II was the minimum site.

(5-3) If the single-end read II could not match either the expression vector sequence or the wild-type genome sequence, it was necessary to assemble and splice the paired-end reads corresponding to the single-end read II into a fragment according to the sequence overlapping characteristics, and ligate the fragment to the T-DNA sequence or inserted expression vector fragment with N to be used as reference sequence, and steps (3) and (5) were repeated until the single-end read II could match the wild-type genome sequence of plants to be tested, and could be determined by step (5-1) or (5-2).

According to the method of step (5), the determination result is shown in Table 2, and the genome schematic diagram of inserted T-DNA sequence is shown in Fig. 5 and Fig. 6.

It can be seen from Fig. 5 and Fig. 6 that this Example belongs to the case of steps (5-1) and (5-3), and the insertion site was located by steps (5-1) and (5-3).

Table2 Final output results of positioning of soybean plants to be tested

| Sample | L22 | L22 |
| --- | --- | --- |
| Vector name | pSOY19-g10-epsps | pSOY19-g10-epsps |
| Vector length | 9557 | 9557 |
| Insertion interval | T-DNA | T-DNA |
| Insertion direction | - | + |
| Insertion sites | 281(right-end of T-DNA) | 6571(left-end of T-DNA) |
| Species genome | Gmax | Gmax |
| Location chromosome | Chr12 | Chr12 |
| Chromosome length | 40091314 | 40091314 |
| Genome insertion position | 32322741 | 32320545 |
| End position | 32323241 | 32320001 |
| Genome chain direction | + | - |

## EXAMPLE 3

(1) Extraction of genomic DNA from experimental materials and plants to be tested: the rice gene editing sample R14 used pHUN4c12S as a vector and had a total length of 12314bp. The target gene for gene editing was OsLCT1 (LOC_Os06g38120), which encoded a low affinity ion transporter.

Firstly, a target sequence (cccggcagcgcaccgatgttgct, the nucleotide sequence was set forth in SEQ ID NO:1) was designed in the third exon of the OsLCT1 gene, and the target sequence was ligated to the vector pHUN4c12S to obtain the pHUN4c12S-OsLCT1 plasmid; then, the plasmid was transformed into Agrobacterium competence. Finally, pHUN4c12S-OsLCT1 was transformed into japonica rice variety Tianjing No.1 by Agrobacterium-mediated transgenic method. After screening, differentiation and rooting, the To transgenic regenerated seedlings were obtained, and the $T_1$ plant R14 was obtained by continuous planting. After transplanting plants to be tested to the field for about 30 days, the leaves were picked and extracted for the genomic DNA of plants to be tested by the conventional DNA extraction kit, the hygromycin-resistant gene marker was used to detect that the plant did not contain hygromycin-resistant gene, and the whole genome re-sequencing was performed. The reference genome sequence of rice was RGAP v7 (http://rice.plantbiology.msu.edu/annotation_pseudo.shtml)

(2) The whole genome re-sequencing was carried out on the genome DNA of plants to be tested, and quality control was carried out on the raw sequencing data to obtain paired-end sequencing data of the whole genome;

the whole genome paired-end sequencing was performed on the rice edited by gene to be tested, transformed with pHUN4c12S by high-throughput sequencer (Illumina Sequencer, Beijing Biomarker Technologies Co., Ltd.), the insertion size was 300bp and the read length was 150bp, 32G raw sequencing data was obtained, and the average sequencing depth was 25 layers.

The raw sequencing data was quality controlled, the software for quality control was Trimmomatic, version 0.36, and criteria of the quality control were:

(2-a) The adapters of sequencing reads were removed;

(2-b) The read with the ratio of N greater than 20% was removed;

(2-c) When the number of low-quality bases (base quality score $\leq 20$) contained at the 3' end of a single-end read (i.e. one of the paired-end reads) exceeded one third of the length of the read, the paired-end reads was removed; parameters were set to tailing: 20, MINLEN: 100, and high quality data after quality control was obtained.

(3) Whether an expression vector sequence containing a T-DNA sequence, inserted in plants to be tested, was known or not was determined, and the following operations according to the determination result were performed:

if the expression vector sequence inserted in the plant to be detected was known, the expression vector containing the T-DNA sequence was used as a reference sequence and the paired-end sequencing data were mapped to the reference sequence to obtain a mapping database;

if the expression vector sequence inserted in the plant to be detected was unknown, a generic vector library was used as reference sequences and the paired-end sequencing data were mapped to the reference sequences to obtain a mapping database;

Here, when the expression vector and T-DNA sequence were unknown, we analyzed them, and then verified them with a known expression vector and T-DNA sequence. The judgemnt result was that the sequence of the expression vector inserted in plants to be tested was unknown, thus the generic vector library was used as reference sequences, and the clean paired-end sequencing data after quality control was mapped and analyzed with the reference sequences to obtain a mapping database.

The specific mapping analysis steps were as follows: the software used was bowtie2, version 2.2.1, the default parameters (the mapping standard had been defined) were adopted, the obtained comparison result information file was in sam format, the sam format file was converted into binary bam format file (i.e. mapping database), and the conversion tool was samtools. In order to reduce the interference of sequencing repetitive sequences (two identical sequences matching the same position and direction of the expression vector) on the subsequent localization results, the step of removing repetitive sequence alignment results was introduced in the format conversion process.

The tool used to remove repetitive sequences was the subroutine module MarkDuplicates.jar in picard software, the parameter REMOVE_DUPLICATES was set to true, and other parameters were set to default.

(4) The read number (FRN) of the matching generic vector library, the average sequencing depth (Gdepth) of the sample genome, the read length (ReadLen) in the mapping database were counted, respectively, and whether transgenic events or gene editing events exist in plants to be tested were determined according to the following formula.

The criteria for determining whether there were transgenic events or gene editing events were (generic vector library):

$$(4\text{-}a)\mathrm{FRN} \geq 125 = (25/2) \times 10;$$

According to the above criteria, it was determined that there were transgenic or gene editing events in the sample.

According to the method of Example 1 combined with Example 3, a total of 78 rice negative control plants, 16 rice gene-edited plants, 5 transgenic rice plants and 9 transgenic soybean materials were tested. There was slight disturbance near the genomic insertion site (case 5-2) in 16 materials during insertion, there was large disturbance or rearrangement near the genomic insertion site (case 5-3) in 5 transgenic materials, there was backbone sequences insertion in 15 materials. A total of 45 insertion events, except for 3 soybean samples which were not verified by PCR, the others were consistent with our analysis results, and the accuracy was 93.7%. The accuracy of this method was 100% when determining whether there were transgenic or gene editing events.

The above is only the preferred embodiment of the present disclosure, and it should be pointed out that for ordinary people in the technical field, several improvements and embellishments can be made without departing from the principle of the present disclosure, and these improvements and embellishments should also be regarded as the protection scope of the present disclosure.

**Claims**

1.  A method for quickly identifying clean transgenic or gene-edited material and insertion sites by using whole genome re-sequencing data, comprising:

    (1) extracting genomic DNA of plant samples to be tested after being processed by transgenic or gene editing technology;
    (2) carrying out whole genome re-sequencing on the genome DNA to obtain paired-end sequencing data of the whole genome;
    (3) determining whether an expression vector sequence containing T-DNA sequence, inserted in plants to be tested, is known or not, and performing the following operations according to the determination result:

       if the expression vector sequence inserted in the plant to be detected is known, the expression vector containing T-DNA sequence is used as a reference sequence and the paired-end sequencing data were mapped to the reference sequence to obtain a mapping database;
       if the expression vector sequence inserted in the plant to be detected is unknown, a generic vector library is used as reference sequences andthe paired-end sequencing data were mapped to the reference sequences to obtain a mapping database;

    (4) counting the read numbers matching expression vector sequences or matching generic vector library in the mapping database, the read numbers matching backbone sequences, the base coverage and average sequencing depth of T-DNA sequences, the average sequencing depth of genome of plant samples to be tested, the sequence length of expression vectors and the length of read, respectively, and determining whether transgenic events or gene editing events exist in plants to be tested, whether backbone sequence transfer events occur, and the copy number of inserted sequences according to the following formula;
    the criteria for determining whether there are transgenic events or gene editing events are:

       (4-1) if the expression vector sequence is known:

$$VRN \geq Gdepth/2 + VectorLen/ReadLen,$$

       and Tcov $\geq$ 0.9; wherein, VRN represents the number of reads matching the expression vector sequence;

Gdepth represents the average sequencing depth of the genome of plants to be tested; VectorLen represents the sequence length of the expression vector; ReadLen represents the length of read; Tcov represents base coverage of T-DNA sequence, Tdepth represents average sequencing depth of T-DNA sequence, and Bdepth represents average sequencing depth of backbone sequence;

(4-2) if the expression vector sequence is unknown:

FRN $\geq$ (Gdepth/2) $\times$ 10, combined with characteristic structure ie., CaMV35S or OsCas9 was identified; wherein, FRN represents the number of reads matching the generic vector library; Gdepth represents the average sequencing depth of the genome of plants to be tested;

the criterion for determining whether there is backbone sequence transfer event is: BRN $\geq$ Gdepth/3; wherein, BRN represents the number of reads matching the backbone sequence; Gdepth represents the average sequencing depth of the genome of plants to be tested;

(4-3) determining copy number: the copy number of inserted T-DNA= Tdepth/Gdepth; the copy number of inserted backbone sequences= Bdepth/Gdepth;

Tdepth represents the average sequencing depth of T-DNA sequence, Gdepth represents the average sequencing depth of the genome of plants to be tested, and Bdepth represents the average sequencing depth of the backbone sequence;

(5) taking the expression vector containing T-DNA sequence as a reference sequence, according to the data of the mapping database, extracting a qualified paired-end reads, wherein the paired-end reads must contain one single-end read I which completely matches the expression vector sequence and another single-end read II which does not match or does not completely match the expression vector sequence; then the single-end read II is compared with the expression vector sequence containing T-DNA sequence and with the wild-type genome sequence of plants to be tested for local homology analysis;

the insertion site of T-DNA sequence is determined according to the following different cases;

(5-1) if one end of the single-end read II can match the expression vector sequence, the other end can match the wild-type genome sequence, and there are at least three single-end reads II with the same matching starting position of genome or expression vector, then it is determined that there is a candidate insertion site on the single-end read II, and the position matching the genome closest to the position of expression vector sequence is the insertion site;

(5-2) if the single-end read II cannot match the expression vector sequence, but can match the wild-type genome sequence, and there are at least three single-end reads II with the same matching starting position of the genome, then it is determined that there is an insertion site on the single-end read II, and the starting position matching the genome is the insertion site;

(5-3) if the single-end read II cannot match either the expression vector sequence or the wild-type genome sequence, then it is necessary to assemble the paired-end reads corresponding to the single-end read II into a fragment according to the sequence overlapping characteristics, and ligate the fragment to the T-DNA sequence or inserted expression vector fragment with N, with the ligated sequence being used as reference sequence, repeating steps (3) and (5) until the single-end read II can match the wild-type genome sequence of plants to be tested and can be judged by step (5-1) or (5-2).

2. The method according to claim 1, wherein in step (2), re-sequencing the whole genome the genomic DNA further comprises performing quality control on the raw sequencing data to obtain clean paired-end sequencing data of the whole genome after quality control;

the quality control comprises the following steps:

(2-a) removing the adapters of sequencing reads;
(2-b) removing the read with the ratio of N greater than 20%;
(2-c) removing the paired-end reads corresponding to the single-end read when the number of low-quality bases contained at the 3' end of the single-end read exceeds one third of the length of the read; the low-quality base is the base having a quality score less than or equal to 20.

3. The method according to claim 1, wherein in step (5-1), the interval sequence in the single-end read II that matches the expression vector sequence is compared with the wild-type genome sequence of the plant samples to be tested; if there is no homologous sequence, then it is determined that the candidate insertion site on the single-end read II is a real insertion site; if there is a homologous sequence, then it is determined that the candidate insertion site on the single-end read II is a false positive insertion site.

4. The method according to claim 1, wherein in step (5-2), if the single-end read II is the left-end sequence of the paired-end reads, then the determined insertion site on the single-end read II is the maximum site; if the single-end read II is the right-end sequence of the paired-end reads, then the determined insertion site on the single-end read II is the minimum site.

5. The method according to claim 1, wherein in steps (5-1)-(5-3), matching criteria are: base similarity ≥95%, mismatched bases ≤5, and base gaps ≤5.

6. The method according to claim 1, further comprising step (6): designing pre-primer and post-primer sequences according to the insertion sites obtained in step (5), and verifying the integrated sites by PCR technology.

**FIG.1**

**FIG.2**

**FIG.3**

**FIG.4**

FIG.5

**FIG.6**

## INTERNATIONAL SEARCH REPORT

| International application No. |
| --- |
| **PCT/CN2020/110191** |

| A. | CLASSIFICATION OF SUBJECT MATTER |
| --- | --- |

C12Q 1/6869(2018.01)i

According to International Patent Classification (IPC) or to both national classification and IPC

| B. | FIELDS SEARCHED |
| --- | --- |

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

CNABS, VEN, CNTXT, WOTXT, USTXT, EPTXT, JPTXT, CNKI, incoPat, Patentics, 百度学术: 基因组, genome, 测序, sequencing, 双末端, pair+ end, 插入, insert+, T-DNA, 转移DNA, transfer DNA

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 110556165 A (ZHEJIANG UNIVERSITY) 10 December 2019 (2019-12-10) Claims 1-6 | 1-6 |
| A | CN 105631242 A (CHINA AGRICULTURAL UNIVERSITY) 01 June 2016 (2016-06-01) entire document | 1-6 |
| A | CN 108034706 A (ZHEJIANG UNIVERSITY) 15 May 2018 (2018-05-15) entire document | 1-6 |
| A | CN 103270175 A (SHENZHEN BGI CO., LTD.) 28 August 2013 (2013-08-28) entire document | 1-6 |
| A | CN 107630079 A (INSTITUTE OF CROP SCIENCE, CHINESE ACADEMY OF AGRICULTURAL SCIENCES) 26 January 2018 (2018-01-26) entire document | 1-6 |
| A | CN 105492625 A (PIONEER HI-BRED INTERNATIONAL, INC.) 13 April 2016 (2016-04-13) entire document | 1-6 |
| A | WO 2013144663 A2 (RUDJER BOSKOVIC INSTITUTE) 03 October 2013 (2013-10-03) entire document | 1-6 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **04 November 2020** | **23 November 2020** |

| Name and mailing address of the ISA/CN | Authorized officer |
| --- | --- |
| **China National Intellectual Property Administration (ISA/CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2020/110191** |

**C.    DOCUMENTS CONSIDERED TO BE RELEVANT**

| Category* | Citation of document, with indication, where appropriate, of the relevant passages | Relevant to claim No. |
| --- | --- | --- |
| A | PARK, D. et al. "A bioinformatics approach for identifying transgene insertion sites using whole genome sequencing data."<br>*BMC Biotechnology.*, Vol. 17, 15 August 2017 (2017-08-15),<br>    article 67, pages 1-8 | 1-6 |

Form PCT/ISA/210 (second sheet) (January 2015)

**INTERNATIONAL SEARCH REPORT**

International application No.

**PCT/CN2020/110191**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

International application No.

**PCT/CN2020/110191**

| Patent document cited in search report | | | Publication date (day/month/year) | Patent family member(s) | | | Publication date (day/month/year) |
|---|---|---|---|---|---|---|---|
| CN | 110556165 | A | 10 December 2019 | None | | | |
| CN | 105631242 | A | 01 June 2016 | CN | 105631242 | B | 11 September 2018 |
| CN | 108034706 | A | 15 May 2018 | None | | | |
| CN | 103270175 | A | 28 August 2013 | WO | 2012097474 | A1 | 26 July 2012 |
| | | | | CN | 103270175 | B | 24 June 2015 |
| CN | 107630079 | A | 26 January 2018 | CN | 107630079 | B | 28 July 2020 |
| CN | 105492625 | A | 13 April 2016 | US | 2018016626 | A1 | 18 January 2018 |
| | | | | MX | 364309 | B | 22 April 2019 |
| | | | | EP | 2986738 | B1 | 24 July 2019 |
| | | | | BR | 112015026499 | A2 | 25 July 2017 |
| | | | | MX | 2015014583 | A | 29 June 2016 |
| | | | | MX | 2019004588 | A | 12 August 2019 |
| | | | | WO | 2014172529 | A1 | 23 October 2014 |
| | | | | CA | 2908361 | A1 | 23 October 2014 |
| | | | | EP | 3578667 | A1 | 11 December 2019 |
| | | | | CN | 111383715 | A | 07 July 2020 |
| | | | | US | 2020032320 | A1 | 30 January 2020 |
| | | | | EP | 2986738 | A1 | 24 February 2016 |
| | | | | US | 2014315726 | A1 | 23 October 2014 |
| | | | | CN | 105492625 | B | 07 April 2020 |
| | | | | US | 10487352 | B2 | 26 November 2019 |
| | | | | US | 9797001 | B2 | 24 October 2017 |
| WO | 2013144663 | A2 | 03 October 2013 | WO | 2013144663 | A3 | 17 April 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910863735X
- CN 103270175 B
- CN 105631242 B

**Non-patent literature cited in the description**

- **HUANG PEITANG et al.** Molecular Cloning: A Laboratory Manual. Science Press, August 2002